# EUROPÄISCHE PATENTSCHRIFT

(11) **EP 2 406 273 B1**
(45) Veröffentlichungstag und Bekanntmachung des Hinweises auf die Patenterteilung: **05.09.2018**
(21) Anmeldenummer: 10713869.5
(22) Anmeldetag: 09.03.2010
(51) Int. Cl.: C07K 1/13, A61K 47/60

(54) **PHOSPHORAMIDAT- UND/ODER PHOSPHONAMIDGRUPPE MODIFIZIERTE MAKROMOLEKÜLE**
PHOSPHORAMIDATE AND/OR PHOSPHONAMIDGROUP MODIFIED MACROMOLECULES
MACROMOLÉCULES MODIFIÉES PAR DES GROUPES PHOSPHONAMIDES ET/OU PAR DES PHOSPHORAMIDATES

(30) Priorität: 09.03.2009 DE 102009012640
(43) Veröffentlichungstag der Anmeldung: 18.01.2012
(73) Patentinhaber: Freie Universität Berlin, 14195 Berlin (DE)
(72) Erfinder: HACKENBERGER, Christian, 14059 Berlin (DE); DEL SIGNORE, Giuseppe, 6511 CM Nijmegen (NL); SERWA, Remigiusz, London NW12JY (GB); WILKENING, Ina, 10713 Berlin (DE); VALLEE, Robert, 14129 Berlin (DE)
(74) Vertreter: Maikowski & Ninnemann Patentanwälte Partnerschaft mbB
(86) Internationale Anmeldenummer: PCT/EP2010/052968
(87) Internationale Veröffentlichungsnummer: WO 2010/102997

(56) Entgegenhaltungen:
- WO-A1-2008/128686
- WO-A2-01/68565
- WO-A2-2006/038184
- WO-A2-2007/110624
- US-A1- 2008 081 782
- CAMP N P ET AL: "Synthesis of stereochemically defined phosphonamidate-containing peptides: Inhibitors for the HIV-1 proteinase", BIOORGANIC & MEDICINAL CHEMISTRY LETTERS, PERGAMON, ELSEVIER SCIENCE, GB, Bd. 2, Nr. 9, 1. September 1992 (1992-09-01), Seiten 1047-1052, XP026762688, ISSN: 0960-894X, DOI: DOI:10.1016/S0960-894X(00)80616-2 [gefunden am 1992-09-01]
- MORR M ET AL: "Cyclic peptide-nucleotide hybrids (cPNH) with phosphoramidate bonds", TETRAHEDRON, ELSEVIER SCIENCE PUBLISHERS, AMSTERDAM, NL, Bd. 55, Nr. 10, 5. März 1999 (1999-03-05), Seiten 2985-2996, XP004157135, ISSN: 0040-4020, DOI: DOI:10.1016/S0040-4020(99)00079-4
- SIKORA D ET AL: "O-Ethyl 1-azidoalkylphosphonic acids-versatile reagents for the synthesis of protected phosphonamidate peptides", TETRAHEDRON, ELSEVIER SCIENCE PUBLISHERS, AMSTERDAM, NL, Bd. 57, Nr. 8, 18. Februar 2001 (2001-02-18), Seiten 1619-1625, XP004317617, ISSN: 0040-4020, DOI: DOI:10.1016/S0040-4020(00)01143-1
- SERWA REMIGIUSZ ET AL.: "Chemoselective Staudinger-phosphite reaction of azides for the phosphorylation of proteins", ANGEWANDTE CHEMIE (INTERNATIONAL ED. IN ENGLISH), Bd. 48, Nr. 44, 27. Juli 2009 (2009-07-27) , Seiten 8234-8239, XP002609595, Germany ISSN: 1521-3773
- BÖHRSCH VERENA ET AL: "Site-specific functionalisation of proteins by a Staudinger-type reaction using unsymmetrical phosphites.", CHEMICAL COMMUNICATIONS (CAMBRIDGE, ENGLAND) 14 MAY 2010 LNKD- PUBMED:20424765, Bd. 46, Nr. 18, 14. Mai 2010 (2010-05-14), Seiten 3176-3178, XP002609596, ISSN: 1364-548X

## Beschreibung

Die vorliegende Erfindung betrifft ein modifiziertes Makromolekül nach Anspruch 1, deren Verwendung nach Anspruch 6, ein Verfahren zur chemoselektiven Phosphorylierung nach Anspruch 7 und ein Verfahren zur ortsspezifischen Immobilisierung nach Anspruch 10, sowie ein Träger herstellbar nach einem Verfahren gemäß Anspruch 10. Chemoselektive Reaktionen sind ein wichtiges Werkzeug in der chemischen Forschung und in den Biowissenschaftlern geworden, da diese einen selektiven Einbau von funktionalen Modulen wie zum Beispiel biophysikalische Sonden in komplexe Biomoleküle ermöglichen. Das wesentliche Element von chemoselektiven Reaktionen ist dabei die selektive Umwandlung einer spezifischen Funktionalität innerhalb eines komplexen Biomoleküls.

Verschiedene Ansätze zur selektiven Funktionalisierung von Biomolekülen, die überwiegend zur Gruppe der Makromoleküle gerechnet werden können, sind bekannt. So können zum Beispiel natürlich auftretende funktionelle Gruppen wie Thiole oder Phenole selektiv modifiziert werden, jedoch ist keine ortsspezifische Markierung möglich, da andere Thiole oder Phenole ebenfalls reagieren können.

Ein weiterer Ansatz besteht in der Einführung von unnatürlichen Funktionalitäten in biologische Moleküle. So ist möglich durch etablierte biochemische Verfahren, Azide in biologische Makromoleküle einzuführen, die einer weiteren Umsetzung zur Verfügung stehen. Dieses Verfahren ermöglicht eine ortsspezifische Modifikation, da die unnatürliche funktionelle Gruppe durch eine chemoselektive Reaktion selektiv funktionalisiert werden kann.

Azid-Gruppen können zum Beispiel mit Molekülen mit Mehrfachbindungen in Gegenwart von Kupfer(I)-Katalysatoren chemoselektiv umgesetzt werden (Klick Chemie), wobei die Verwendung von toxischen Kupfer(I)-Katalysatoren für die Anwendung in lebenden Systemen nachteilig ist.

Ein anderer Ansatz wird in der WO 2001/068565 A2 oder DE 601 20 650 beschrieben. Hier erfolgt eine chemoselektive Ligationsreaktion unter Verwendung von Phosphinen zur Verknüpfung von zwei Reaktionspartnern unter milden Reaktionsbedingungen (Staudinger-Ligation). Diese Ligationsreaktion basiert auf der Staudinger Reaktion, in der ein Azid chemoselektiv mit einem Phosphin zu einem Iminophosphoran reagiert. Um eine Hydrolyse des Iminophosphorans zu vermeiden, ist diese Reaktion dahingehend manipulierbar, dass ein interner elektrophiler Substituent des Phosphins das entstehende Iminophosphoran abfängt, wodurch es zur Bildung einer Amidbindung im Endprodukt kommt. Als nachteilig erweist sich in diesem Prozess die langsame Reaktionsgeschwindigkeit, die immer noch auftretende kompetitive Iminophosphoran-Hydrolyse, die Oxidationsempfindlichkeit von Phosphinen und die Tatsache, dass die Phosphine mit dem internen elektophilen Substituenten über einen mehrstufigen Prozess hergestellt werden müssen.

Der Erfindung liegt daher das Problem zu Grunde, ein chemoselektives Verfahren zur Herstellung modifizierter Verbindungen, insbesondere von modifizierten Makromolekülen zur Verfügung zu stellen, welche die oben genannten Nachteile nicht aufweist.

Diese Aufgabe wird erfindungsgemäß durch die Bereitstellung eines modifizierten Makromoleküles, mit den Merkmalen des Anspruchs 1 gelöst.

Danach ist das modifizierte mindestens eine weitere funktionelle Gruppe aufweisendes Makromolekül durch mindestens eine x-fach (x≥1) chemoselektiv eingebaute Phosphoramidat-Gruppe der allgemeinen Formel (I)

-NPO(OR¹)(OR^{1'}) (I)

und/oder mindestens eine x-fach (x≥1) chemoselektiv eingebaute Phosphonamid-Gruppe der allgemeinen Formel (Ia)

-NPO(R¹)(OR¹) (Ia)

gekennzeichnet, wobei R¹ und R^{1'} ausgewählt ist aus der Gruppe enthaltend
- Glycerol, Polyglycerol,
- PEG-Polymere der allgemeinen Summenformel C₂ₙH₄ₙ₊₂Oₙ₊₁ mit n ≥ 1
- Cₙ-Alkylketten mit n ≥ 1, bevorzugt Methyl, Ethyl oder Butyl,
- funktionalisierte Cₙ-Alkylketten mit n ≥ 1, bevorzugt -CH₂-(NO₂)Ar, Coumarine, enzymatisch spaltbare Ester, 2-Oxo-prop-1-yl, 3-Oxo-but-1-yl, Cyanomethyl, Nitromethyl, Chlormethyl, Hydroxymethyl, Tetrahydropyranyloxymethyl, Trityloxymethyl, Propionyloxymethyl, Aminomethyl, Carboxymethyl, Allyloxycarbonylmethyl, Allyloxycarbonylaminomethyl, Methoxymethyl, Ethoxymethyl, t-Butoxymethyl, Acetoxymethyl, Chlormethyl, Brommethyl, Idmethyl, Trifluormethyl, 6-Hydroxyhexyl, 2,4-Dichlor(n-butyl), 2-Aminopropyl, 1-Chlorethyl, 2-Chlorethyl, 1-Bromethyl, 2-Bromethyl, 1-Fluorethyl, 2-Fluorethyl, 1-lodethyl, 2-lodethyl, 1-Chlorpropyl, 2-Chlorpropyl, 3-Chlorpropyl, 1-Brompropyl, 2-Brompropyl, 3-Brompropyl, 1-Fluorpropyl, 2-Fluorptopyl, 3-Fluorpropyl, 1-lodpropyl, 2-lodpropyl, 3-Iodpropyl, 2-Aminoethyl, 1-Aminoethyl, N-Benzoyl-2-Aminoethyl, N-Acetyl-2-Aminoethyl, N-Benzoyl-1-Aminoethyl oder N-Acetyl-1-Aminoethyl,
- Aryle, bevorzugt Phenyl, Benzyl, Naphthyl, oder Anthryl.
- Heteroaryl, bevorzugt Pyridinyl-, Pyrimidinyl, Pyrazinyl, Pyridazinyl, Pyrrolo, Furano, Oxazolo, Isooxazolo, Phthalimido, Thioazolo
- Silyl, bevorzugt Trialkylsilyl,
- Lipide, bevorzugt hydrophobe Fettsäurereste, Terpene wie Palmitoyl oder Farnesyl,
- Fluorophore, bevorzugt Fluorescin, Bodipy-Farbstoffe oder FRET-Label
- Isotopenlabel, bevorzugt radioaktive Isotope, Isotopen für NMR-Verfahren oder Imaging-Verfahren,
- Saccharide, insbesondere Oligosaccharide,
- Peptide,
- Kronenether, oder
- ein Linker, der die oben angegebenen Gruppen verknüpft,
und wobei R¹ und R^{1'} gleich oder verschieden voneinander sein können, und wobei die mindestens eine weitere funktionelle Gruppe ausgewählt ist aus der Gruppe enthaltend -OH, -NH₂, -SH, -CO₂H, -CONH₂, sowie Disulfide, Guanidine, Imidazole, Indole, Alkene und/oder Alkine. Die Anzahl n der eingebauten Phosphoramidat- und/oder Phosphonsäuregruppen ist dabei aufgrund des angewandten Syntheseverfahrens gemäß der Staudinger-Reaktion ausgehend von funktionalisierten Aziden in definierter Weise gezielt, d.h. chemoselektiv, einstellbar. Es erfolgt also keine willkürliche und ortsunspezifische Modifizierung von Makromolekülen. Aufgrund der Bedingungen der chemoselektiven Synthese sind die Phosphoramidat- und/oder Phosphonsäuregruppen bevorzugt an aromatische und/oder alkylische Reste der Bio- oder Makromoleküle gebunden. So erfolgt die chemoselektive Modifizierung von Proteinen und/oder Peptiden bevorzugt über die Verwendung von p-Azidophenylalanin oder verschiedene Alkylazidoaminosäuren wie z.B. Azidohomoalanin. Somit sind die Phosphoramidat- und/oder Phosphonsäuregruppen in Proteinen insbesondere an einen Phenylalanin- und/oder Homoalanin-Rest gebunden. Bevorzugt werden für die Modifizierung der Makromoleküle also Alkyl- bzw. Arylazide verwendet.

Die oben angeführten Gruppen R¹, R^{1'} und R^{1"} können selbstverständlich in substituierter oder unsusbstituierter Form vorliegen, wobei mögliche Substituenten dem Fachmann geläufig sind. Bevorzugt eingesetzte Gruppen sind Fluorophore, wie Fluorescin, Bodipy-Farbstoffe oder FRET-Label, Isotopenlabel, Saccharide, Nitrobenzyl, PEG's, Coumarin, Biotin. Besonders bevorzugt ist die Verwendung von Fluorophoren, PEG's, Biotin und/oder Isotopenlabels.

Bevorzugt ist somit über die Phosphoramidatgruppe und/oder die Phosphonamidgruppe eine funktionelle Gruppe an die höhermolekularen Verbindungen gekoppelt.

Bevorzugt liegt die weitere funktionelle Gruppe des modifizierten Makromoleküls, geschützt oder ungeschützt vor. Besonders bevorzugte funktionelle Gruppen sind ausgewählt aus einer Gruppe enthaltend -OH, -NH₂, -SH, -CO₂H,-CONH₂ sowie Disulfide, Guanidine wie -NHCNHNH₂ in Arginin, Imidiazole wie in Histidin, Indole wie in Tryptophan. Zusätzlich können in Makromolekülen als weitere funktionelle Gruppen Alkene und/oder Alkine vorliegen,

Die Anzahl x der eingebauten Phosphoramidat- und/oder Phosphonsäuregruppen liegt bevorzugt zwischen 1 und 100, besonders bevorzugt zwischen 1 und 30 und ganz besonders bevorzugt zwischen 1 und 15.

Die Herstellung der erfindungsgemäßen Verbindungen erfolgt in einer modifizierten Staudinger- Reaktion, wobei die Verbindungen bei einem pH-Wert zwischen 6 und 10, bevorzugt bei pH 7 bis 8, bei Temperaturen zwischen 18 und 45°C, bevorzugt bei Raumtemperatur, für einen Zeitraum bis zu 48 h, bevorzugt 12 h mit einem Phosphit der allgemeinen Formel (II)

P(OR¹)(OR^{1'})(OR^{1"}) (II),

und/oder einem Phosphonit der allgemeinen Formel (IIa)

PR¹(OR')(OR^{1"}) (IIa)

wobei R¹, R^{1'} und R^{1"} die obige Bedeutung von R¹ und R^{1'} aufweisen, und gleich oder verschieden voneinander sein können, umgesetzt werden.

Umsetzungen sind jedoch auch in oder durch Zusatz von anderen Lösungsmitteln, bevorzugt DMF; DMSO, THF, durchführbar, sofern die zu modizierenden Verbindungen das zugesetzte Lösungsmittel tolerieren.

Das hier beschriebene chemoselektive Verfahren ermöglicht die Durchführung der Kopplungsreaktion unter milden Reaktionsbedingungen, z.B. in wässriger Lösung bei Raumtemperatur. Die Phosphit- und Phosphonitkomponenten sind synthetisch einfach herstellbar und nicht oxidationsempfindlich. Darüber hinaus benötigt die chemoselektive Reaktion zwischen Aziden und Phosphiten und/oder Phosphoniten nicht die Zugabe von toxischen Cu(I)-Metallkatalysatoren wie z.B. bei Anwendung der Klick-Chemie. Eine Anwendung in einer intrazellulären Reaktion ist daher möglich.

In einer Ausführungsform der vorliegenden Erfindung ist die in das Makromolekül eingebaute modifizierte Phosphoramidatgruppe der Formel (I) in eine freie Phosphoramid-Gruppe der allgemeinen Formel (III)

-NHPO(OH)₂ (III)

photolytisch und/oder hydrolytisch umwandelbar. Dies ermöglicht die Herstellung von Analoga von phosphorylierten Proteinen, die genauso wie phosphorylierte Proteine z.B. von Antikörpern oder Kinasen erkennbar sind.

Ebenso ist die eingebaute modifizierte Phosphonamidgruppe der Formel (la) in eine freie Phosphonamid-Gruppe der allgemeinen Formel (IIIa)

-NHP(O)R¹(OH) (IIIa)

photolytisch und/oder hydrolytisch umwandelbar.

So ist es möglich, durch Abspaltung von photolabilen Gruppen wie *o*-Nitrobenzyl oder Coumarine die Verbindungen zu Verbindungen der allgemeinen Formel (III) oder (IIIa) zu gelangen.

In einer weiteren Ausführungsform sind die erfindungsgemäßen Verbindungen über die eingebauten Phosphoramidat / Phosphonamidgruppen auf der Oberfläche eines Trägers immobilisierbar. Als Träger sind hier funktionalisierte Glasoberflächen, Mikroplatten, Nanopartikel, insbesondere Gold oder Silicananopartikel und Quantum Dots verwendbar.

Die zum Einsatz kommenden Verbindungen sind bevorzugt ausgewählt aus der Gruppe enthaltend Polymere, Peptide, Proteine, Glykane, Lipide, Polynukleotide, Polyketide und/oder andere Naturstoffe. Wichtig ist, dass die zu modifizierenden Makromoleküle mindestens eine Azid-Gruppe aufweisen, die z.B, während des Syntheseprozesses in das Molekül entsprechend eingeführt wird.

Als Peptide werden Peptide mit einer Kettenlänge von größer als 2 Aminosäuren, insbesondere größer als 6 Aminosäuren verwendet. Von besonderem Interesse sind Peptide und Hormone von pharmakologischer Relevanz, die zur Verbesserung der biologischen Aktivität oder Bioverfügbarkeit modifiziert werden sollen. Die Hormone und/oder Peptide können ausgewählt sein aus einer Gruppe enthaltend Wachstumshormone, Stresshormone, Steroidhormone, Neuropeptide, Schilddrüsenhormone, Peptidhormone des Magen-Darmtraktes und andere.

Als Polymere können Polyglycerole, insbesondere PG10 oder andere Polymere, die eine Azidgruppe tragen, verwendet werden. Die verwendeten Polymere weisen typischerweise eine Molmasse zwischen 1 und 40 kDa auf.

Besonders vorteilhaft ist die Modifikation von physiologisch relevanten Naturstoffen wie Signalpeptiden, Glycolipiden oder Sphingolipiden oder pharmakologisch und biologisch relevanten Proteinen, die insbesondere für die Proteomforschung und die Aufklärung von Signaltransduktionsprozessen von Bedeutung sind.

Eine besonders interessante Gruppe von Proteinen stellen dabei Proteinklassen dar, die für den Transport von Proteinen aus dem Zellinneren über die Membran verantwortlich sind. Ein typischer Vertreter dieser Klasse ist das Sec-System umfassend z.B. SecB. Ebenfalls sind Kinasen involviert in Signaltransduktion oder in Zuckerstoffwechsel bevorzugt wie z.B. Glucokinasen, Phosphofructokinasen oder Proteinkinasen. Eine weitere bevorzugte Gruppe an Proteinen umfasst die Klasse der Phosphatasen, regulatorischen Proteine und/oder Glykoproteine.

Die erfindungsgemäßen Verbindungen können in der pharmazeutischen oder biotechnologischen Industrie als Phosphatase- oder Kinasesubstrate, als Markermoleküle, als biochemische Sonden zur Aufklärung biologischer Funktion, als Stabilisierungsfaktoren gegenüber Proteolyse durch den Einbau von PEG oder auch als pharmazeutisch wirksame Substanzen, insbesondere Inhibitoren verwendet werden.

Eine ortsspezifische Markierung bzw. Funktionalisierung ermöglicht z.B. eine genaue Analyse der Funktion, insbesondere in komplexer nativer Umgebung. Des Weiteren ist aufgrund der Immobiliserung der modifizierten Verbindungen eine Anwendung in der Wirkstoffforschung möglich, da kleine Moleküle in einem Screeningverfahren getestet werden können. Ebenso könnte die Phosphoramidateinheit selbst auch eine biologische Wirkung haben, so dass in Kombination mit Lipiden oder Peptiden der Einsatz als Enzyminhibitoren möglich ist.

Ein großes Anwendungsgebiet ist die Biokonjugation von PEG-Polymeren. PEG dient als Stabiliserung des Proteins gegenüber ungewolltem Proteinabbau (Proteolyse). Eine alternative und ortsspezifische Biokonjugation von PEG ist von großem Interesse für die Industrie, da Proteine so bioverfügbar (bioavailable) gemacht werden können. Die verwendeten PEGs weisen bevorzugt eine mittlere Molmasse von 100 bis 40.000 auf.

Die Aufgabe der vorliegenden Erfindung wird auch durch Verfahren mit den Merkmalen der Ansprüche 7 und 10 gelöst.

Mittels des chemoselektiven Verfahrens unter Verwendung von Phosphit und/oder Phosphonit ist die Herstellung von modifizierten Proteinen oder modifizierten Proteinanaloga, insbesondere von Glycoproteinen möglich. Das Verfahren zur chemoselektiven Phosphorylierung bzw. Phosphonylierung von Verbindungen umfasst dabei die folgenden Schritte:
a) Synthese und/oder Funktionalisierung einer Verbindung mit mindestens einer Azid (N₃) - Gruppe, bevorzugt mit einer Alkyl- und/oder Arylazidgruppe, und
b) Umsetzung der mindestens einen Azid-Gruppe der Verbindung
   - mit einem Phosphit der allgemeinen Formel (II) P(OR¹)(OR^{1'})(OR^{1"}) unter Ausbildung einer Phosphoramidat-Gruppe der allgemeinen Formel (I) -NHPO(OR¹)(OR^{1'}) und/oder
   - mit einem Phosphonit der allgemeinen Formel (IIa) PR(OR^{1'})(OR^{1"}) unter Ausbildung einer Phosphonamid-Gruppe der allgemeinen Formel (Ia) -NHPO(R1)(OR1'), wobei den Resten R¹, R^{1'} und R^{1"} die obige Bedeutung von R¹ und R^{1'} zukommt.

Für die vorliegende Erfindung wesentlich ist, dass das verwendete Azid mindestens eine weitere funktionelle Gruppe im Molekül aufweist. Dies ist bei der Mehrzahl der zum Einsatz kommenden und zu modifizierenden Bio- oder Makromoleküle der Fall. Die weitere funktionelle Gruppe kann geschützt oder ungeschützt vorliegen. Besonders bevorzugte funktionelle Gruppen sind ausgewählt aus einer Gruppe enthaltend -OH, -NH₂, -SH, -CO₂H, - CONH₂, sowie Disulfide, Guanidine wie -NHCNHNH₂ in Arginin, Imidiazole wie in Histidin, Indole wie in Tryptophan. Zusätzlich können in Makromolekülen als weitere funktionelle Gruppen Alkene und/oder Alkine vorliegen.

In einer bevorzugten Variante wird in einem weiteren Schritt c) die Phosporamidatgruppe der allgemeinen Formel (I) zu einer Phosphoramid-Gruppe der allgemeinen Formel (III)--NHPO(OH)₂ und/oder die Phosphonamidgruppe der allgemeinen Formel (la) zu einer Phosphonamid-Gruppe der allgemeinen Formel (lIla) -NHPO(R₁)OH umgesetzt.

Der Schritt der Freisetzung der Phosporamidat- und/oder Phosphonamid-Gruppe erfolgt bevorzugt photolytisch und/oder hydrolytisch.

Die Umsetzung der N₃-haltigen Verbindung mit einem Phosphit der allgemeinen Formel (II) P(OR¹)(OR^{1'})(OR^{1"}) und/oder einem Phosphonit der allgemeinen Formel (IIa) PR¹(OR^{1'})(OR^{1"}) wird bevorzugt bei einem pH-Wert zwischen 6 und 10, bevorzugt bei 7 bis 8, bei Temperaturen zwischen 18 und 45°C, bevorzugt bei Raumtemperatur, für einen Zeitraum bis zu 48 h, bevorzugt 12 h, durchgeführt.

Die mittels dieses Verfahrens hergestellten Verbindungen, insbesondere die phosphorylierten Proteine, stellen Analoga zu natürlich phosphorylierten Proteinen insbesondere mit Phosphorylierungen an den Aminosäuren Ser, Thr und Tyr dar und sind z.B. durch Antikörper, Kinasen oder Phosphatasen oder Proteindomänen, wie z.B. SH₂ Domänen erkennbar.

Das chemoselektive Verfahren ist ebenfalls zur ortsspezifischen Immobilisierung von Verbindungen auf Trägern anwendbar. Das Verfahren zur ortsspezifischen Immobilisierung von Verbindungen umfasst die folgenden Schritte
a) Synthese oder Funktionalisierung einer Verbindung mit mindestens einer Azid (N₃₋) Gruppe, bevorzugt mit einer Alkyl- und/oder Arylazidgruppe,
b) Kopplung von mindestens einem Phosphit der allgemeinen Formel (IV) P(OR²)(OR^{2'})(OR^{2"}) und/oder mindestens einem Phosphonit der allgemeinen Formel (IVa) PR²(OR^{2'})(OR^{2"}) an die Oberfläche von mindestens einem Träger R³, wobei R², R^{2'} und R^{2"} ausgewählt sind aus der Gruppe enthaltend Cₙ-Alkylketten mit n ≥ 1, bevorzugt Methyl, Ethyl oder Butyl, funktionalisierte Cₙ-Alkylketten mit n ≥ 1, bevorzugt -CH₂-(NO₂)Ar, Coumarine, enzymatisch spaltbare Ester, 2-Oxo-prop-1-yl, 3-Oxo-but-1-yl, Cyanomethyl, Nitromethyl, Chlormethyl, Hydroxymethyl, Tetrahydropyranyloxymethy, Trityloxymethyl, Propionyloxymethyl, Aminomethyl, Carboxymethyl, Allyloxycarbonylmethyl, Allyloxycarbonylaminomethyl, Methoxymethyl, Ethoxymethyl, t-Butoxymethyl, Acetoxymethyl, Chlormethyl, Brommethyl, Idmethyl, Trifluormethyl, 6-Hydroxyhexyl, 2,4-Dichlor(n-butyl), 2-Aminopropyl, 1-Chlorethyl, 2-Chlorethyl, 1-Bromethyl, 2-Bromethyl, 1-Fluorethyl, 2-Fluorethyl, 1-lodethyl, 2-lodethyl, 1-Chlorpropyl, 2-Chlorpropyl, 3-Chlorpropyl, 1-Brompropyl, 2-Brompropyl, 3-Brompropyl, 1-Fluorpropyl, 2-Fluorptopyl, 3-Fluorpropyl, 1-lodpropyl, 2-lodpropyl, 3-lodpropyl, 2-Aminoethyl, 1-Aminoethyl, N-Benzoyl-2-Aminoethyl, N-Acetyl-2-Aminoethyl, N-Benzoyl-1-Aminoethyl oder N-Acetyl-1-Aminoethyl; Aryle, bevorzugt Phenyl, Benzyl, Naphthyl, oder Anthryl; Heteroaryl, bevorzugt Pyridinyl-, Pyrimidinyl, Pyrazinyl, Pyridazinyl, Pyrrolo, Furano, Oxazolo, Isooxazolo, Phthalimido, Thioazolo; Silyl, bevorzugt Trialkylsilyl, wobei R², R^{2'} und R^{2"} gleich oder verschieden voneinander sein können, und
c) Umsetzung der mindestens einen Azid-Gruppe der Verbindung mit dem mindestens einen mit dem Phosphit und/oder Phosphonit gekoppelten Träger, wobei eine Kopplung zwischen dem Träger und der Verbindung über eine gebildete Phosphoramidat- und/oder Phosphonamidgruppe erfolgt.

Die oben angeführten Gruppen können selbstverständlich in substituierter oder unsusbstituierter Form vorliegen, wobei mögliche Substituenten dem Fachmann geläufig sind.
Wesentlich ist auch hier, dass das verwendete Azid mindestens eine weitere funktionelle Gruppe im Molekül aufweist. Dies ist bei der Mehrzahl der zum Einsatz kommenden und zu modifizierenden Makromolekülen der Fall. Die weitere funktionelle Gruppe kann geschützt oder ungeschützt vorliegen. Besonders bevorzugte funktionelle Gruppen sind ausgewählt aus einer Gruppe enthaltend -OH, -NH₂, -SH, -CO₂H, -CONH₂, sowie Disulfide, Guanidine wie -NHCNHNH₂ in Arginin, Imidiazole wie in Histidin, Indole wie in Tryptophan. Zusätzlich können in Makromolekülen als weitere funktionelle Gruppen Alkene und/oder Alkine vorliegen
Als bevorzugte Trägermaterialien kommen Glas, Polymer, Silica oder Nanopartikel zum Einsatz. Besonders bevorzugt Trägermaterialien sind Gold, Quantum Dots, Silica und Glasoberflächen.
Die Kopplung an die Oberfläche des Trägermateriales erfolgt bevorzugt somit entweder über eine Phosphitbindung der allgemeinen Formel R³O-P(OR²)(OR²) oder über eine Phosphonitbindung der allgemeinen Formel R³-P(OR²)(OR^{2'}) erfolgen, wobei R³, die Verbindung zur Oberfläche darstellt. Die ortsspezifische Immobiliserung an verschiedene Oberflächen und Träger erfolgt unter analogen Reaktionsbedingungen wie zuvor beschrieben. Dabei wird ein Phosphit-funktionalisierter Träger mit einer Lösung der Azidohaltigen Verbindung behandelt, wobei es zur Ausbildung einer kovalenten Verknüpfung zwischen der Verbindung und dem Träger kommt.

Mit diesem Verfahren können insbesondere Biomoleküle für Hochdurchsatzverfahren (Screening) immobilisiert werden. Besonders wichtig ist dabei, dass eine ortsspezifische Immobiliserung ein natives Verhalten des Biomoleküls in Lösung ermöglicht, so dass die Ergebnisse des Hochdurchsatzverfahrens den nativen Bedingungen sehr nahe kommt.

Die Erfindung wird nachfolgend unter Bezugnahme auf die Figuren anhand von verschiedenen Ausführungsbeispielen näher erläutert.

Es zeigen
- Figur 1: Biokonjugation mittels der Staudinger-Phosphit Reaktion;
- Figur 2: Staudinger-Phosphit Reaktion von ungeschützten Phenyl-Azido-Peptiden;
- Figur 3A: Darstellung eines Phosphoramidat-haltigen Proteins durch die Staudinger-Phosphit Reaktion;
- Figur 3B: a) Aminosäuresequenz modifiziertes SecB (X: p-azido-Phe), b) zu erwartende Aminosäuresequnzen der proteolytischen Spaltung mit GluC bzw. AspN (Y: phosphorylierte Aminosäure);
- Figur 3C: Chemoselektive Phosphorylierung von Biomolekülen;
- Figur 3D: Staudinger-Phosphit-Reaktion mit Azido-SecB und Gelelektrophorese;
- Figur 3E: Antikörper-Erkennung entschützter Phosphoramidate;
- Figur 4: Chemoselektive Protein-Immobiliserung durch die Staudinger-Phosphit Reaktion;
- Figur 5: ¹H-NMR Spektrum eines Polyglycerol-Methylphosphoramidat-Derivates;
- Figur 6: Modifizierung eines Peptides mittels der erfindungsgemäßen Reaktion;
- Figuren 7A-C: Modifizierung eines Proteines mit einem ersten PEG-Molekül mittels der erfindungsgemäßen Reaktion;
- Figuren 8A-B: Modifizierung eines Proteines mit einem zweitem PEG-Molekül mittels der erfindungsgemäßen Reaktion; und
- Figur 9: Modifizierung eines Proteines mit Biotin mittels der erfindungsgemäßen Reaktion.

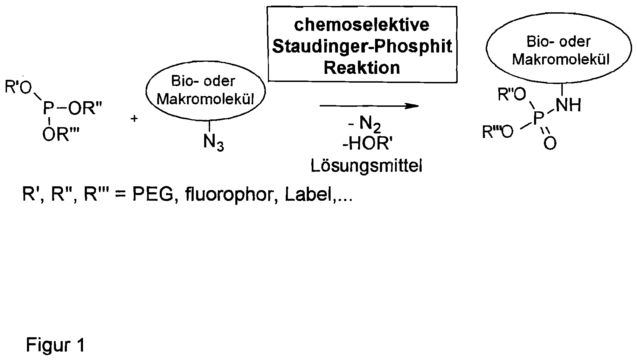

Figur 1 zeigt ein allgemeines Schema zur ortsspezifischen Funktionalisierung von Biomolekülen mittels der modifizierten Staudinger-Phosphit Reaktion. Es lassen sich verschiedene modifizierte Verbindungen, insbesondere Biomoleküle (Biokonjugate) herstellen. So können Biokonjugate mit PEG-Polymeren, langen Alkylketten, Fluorophoren oder Isotopenlabel hergestellt werden. Die Reaktion erfolgt in wässrigen Lösungsmitteln unter milden Reaktionsbedingungen.

### Ausführungsbeispiel 1: Darstellung modifizierter Peptide

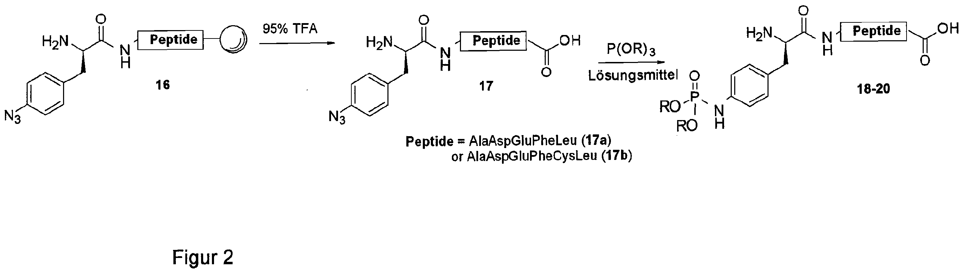

Figur 2 zeigt die chemoselektive Synthese von mit Phosphoramidat-Gruppen modifizierter Peptide AlaAspGluPheLeu **17a** und AlaAspGluPheCysLeu **17b** (siehe Tabelle 1). Hierfür werden die Peptide auf einem ABI443A Petidsynthesizer unter Verwendung der Standardbedingungen zur Amidkopplung gemäß dem HBTU/HOBt (Fast-Fmoc-Protokoll) auf einem Wang-Harz mit Fmoc-p-azido-Phe-OH als letztem Rest synthetisiert. Die Peptide werden von der Festphase mit 95% TFA abgespalten und mittels semi-präperativer HPLC aufgereinigt.

Anschließend werden zu einer Lösung der Azido-Peptide (0,2 ml/mg Peptid) 5-10 Äquivalente Phosphit (siehe Tabelle 1) gegeben und die Reaktionslösung wird bei Raumtemperatur für 6 bis 24 h gerührt. Ohne weitere Aufarbeitung werden die Phosphoramidat-Peptide **18-20** aus der Reaktionslösung mittels präperativer HPLC isoliert und nachfolgend lyophilisiert. Die isolierten Ausbeuten liegen zwischen 39 und 63 % (siehe Tabelle 1).

**Tabelle 1: Modifizierte Peptide**

| Nr | Peptide | Phosphite | Lösungs mittel | Produkt | Zeit [h] | Ausbeute[%] |
|---|---|---|---|---|---|---|
| 1 | **17a** | P(OBu)₃ | DMSO | **18** | 24 | 63 |
| 2 | **17a** | P[(OCH₂CH₂)₄OCH₃]₃ | CH₃CN | **19** | 24 | 61 |
| 3 | **17b** | P(OEt)₃ | DMSO | **20** | 6 | 39 |

### Ausführungsbeispiel 2: Darstellung phosphorylierter Proteine

### 2.1. Staudinger-Phosphit Reaktion von Azidoproteinen mit Trimethyl-phosphit

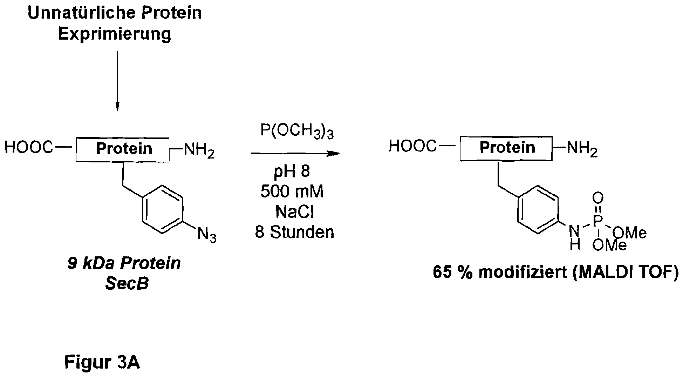

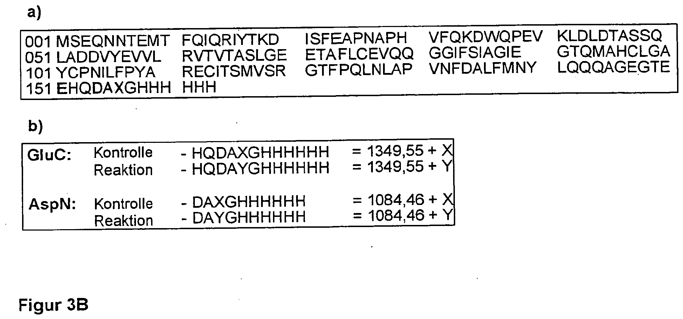

Die chemoselektive Funktionalisierung von Azido-Proteinen mit einer Phosphoramidateinheit durch eine Staudinger-Phosphit Reaktion wurde durch das folgende Experiment demonstriert. Dafür wurde ein Modellprotein, das SecB, mit einer p-azido-Phe-Aminosäure exprimiert (siehe Figur 3A,B).

Anschließend wurde das Protein, gelöst in einem Imidazol-Puffer bei pH 8, mit Trimethylphosphit versetzt und über Nacht bei Raumtemperatur bzw. 37 °C geschüttelt. Die Aufreinigung und Charakterisierung erfolgte über Gelelektrophorese und MALDI-MS, wobei zusätzlich zu den beiden Reaktionslösungen auch eine Lösung des Ausgangsproteins zum direkten Vergleich gemessen wurde.

Da das MALDI-Massenspektrometer nur Massen von 800 bis 3000 (m/z) detektieren kann, wurde das nachzuweisende Protein zunächst proteolytisch gespalten. Da sich in Nachbarschaft zum N-Terminus von p-azido-Phe jeweils eine Glutamin- und Asparaginsäure, wurde eine Protease-Spaltung mit GluC bzw. AspN durchgeführt. Die dabei zu erwartenden Sequenzen mit den dazu gehörigen Masse-Ladungsverhältnissen sind in Figur 3B b) dargestellt. Die im MALDI-Massenspektrometer erhaltenen Messwerte sind in Tabelle 2 zusammengefasst.

**Tabelle 2: MALDI-MS-Messwerte nach proteolytischer Spaltung**

| Protease | theor. Masse (ohne X/Y) | Messwert (mit X) | Masse X | Messwert (mit Y) | Masse Y |
|---|---|---|---|---|---|
| GluC | 1349,55 | 1511,65 | 162,10 | 1619,64 | 270,09 |
| AspN | 1084,46 | 1246,50 | 162,04 | 1354,52 | 270,06 |

In beiden Messungen war nach Reaktion mit Trimethylphosphit neben einer Intensitätsabnahme des Ausgangspeaks ein neuer Peak bei höherem Masse-Ladungsverhältnis zu verzeichnen. Die erhaltenen Massendifferenzen für X und Y passen auf die erste Nachkommastelle genau zu den in Figur 3B dargestellten Strukturen des reduzierten Amins (X) bzw. der phosphorylierten Aminosäure (Y). Das Verhältnis der beiden Massen wurde auf 35 zu 65 (X zu Y) bestimmt, so dass eine 65%ige Modifizierung des Azido-Proteins zum Phosphoramidat erreicht wurde. Somit konnte das phosphorylierte Protein eindeutig nachgewiesen werden, was demonstriert, das die Reaktion auch im wässrigen Milieu bei sehr komplexen Proteinen funktioniert.

### 2.2. Analoga phosphorylierter Proteine durch die Staudinger-Phosphit Reaktion

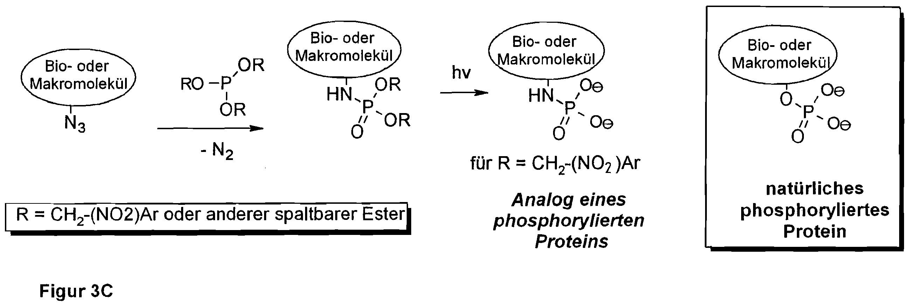

Basierend auf den Ergebnissen aus 2.1. wurden im folgenden Experiment Protein-Phosphoramidate hergestellt, die nach photolytischer Spaltung in die entsprechenden Phosphatanaloga überführt wurden (Figur 3C).

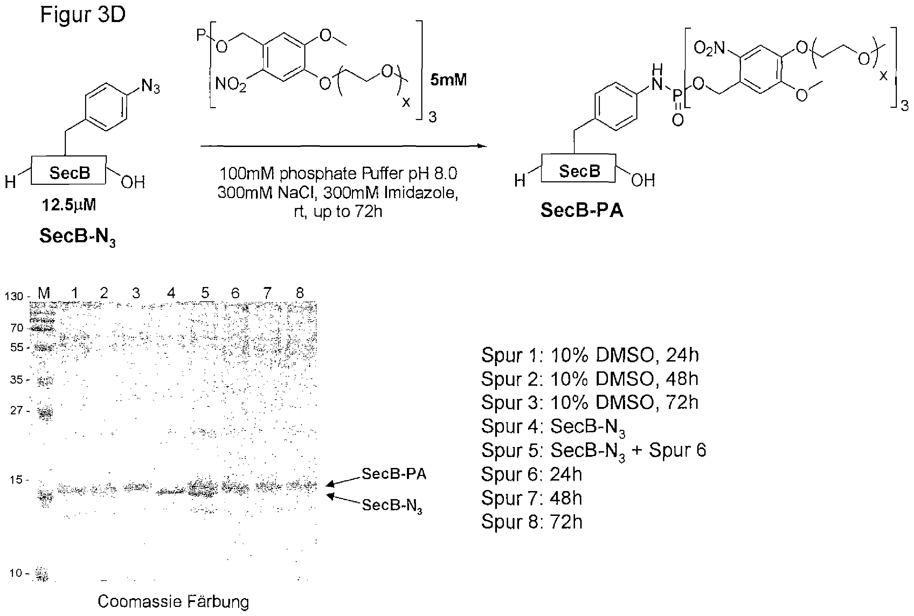

In einer Modellstudie wurde auch hierfür das in 2.1. beschriebene Azido-SecB Protein verwendet. Dieses wurde mit einem wasserlöslichen Phosphit umgesetzt, das drei photolabile Ester trägt (Figur 3D).

Eine durchgeführte Gelelektrophorese zeigte, dass das Protein nach 24h, sowohl in reinem Puffer bei pH 8 als auch unter Zusatz von 10% DMSO vollständig in das Phosphoramidat überführt wurde (Figur 3D).

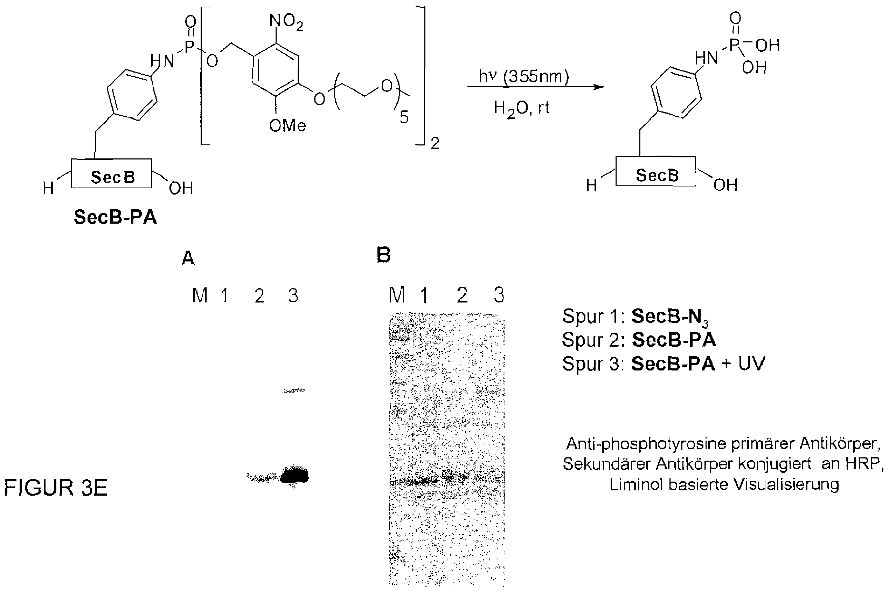

Anschließend wurde das Phosphoramidat-Protein unter Bestrahlung bei 355nm photolysiert und so in das geladene Phosphroamidat überführt (Figur 3E).

Anschließend wurden Erkennungsreaktionen mit einem Antikörper durchgeführt, der normalerweise phosphorylierte Tyr erkennt (Anti-phosphotyrosin Antikörper). Es zeigte sich durch Western Blot Analyse (Gel A, Figur 3E), dass nach Photolyse (Lane 3) eine eindeutige Erkennung des Antikörpers (tiefschwarze Verfärbung) stattfindet. Eine schwache Erkennung des geschützten Phosphoramidates (Lane 2) ist auf eine teilweise Photolyse durch normales Tageslicht zurückzuführen. Unmodiziertes Azido-Sec B (Lane 1) wird dagegen nicht erkannt. Zusammenfassend kann daher festgehalten werden, dass die Staudinger Phosphit Reaktion zur quantitativen Darstellung von Phosphoramidat-haltigen Proteinen verwendet werden kann. Diese Proteine können durch geeignete Esterspaltungen in die geladenen Phosphoramidate überführt werden, die in Antikörperreaktionen das gleiche Verhalten wie natürlich phosphorylierte Proteine (in diesem Fall durch Phosphorylierung eines Tyrosins) zeigen.

### Ausführungsbeispiel 3: Immobilisierung einer Verbindung

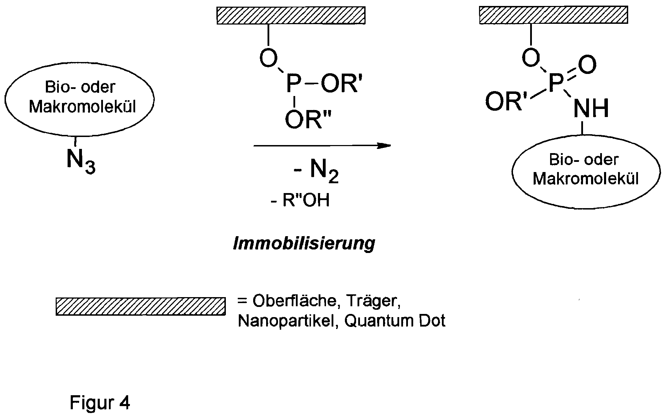

Figur 4 zeigt die Anwendung der Staudinger-Phosphit-Reaktion zur ortsspezifischen Immobilisierung von Verbindungen. Die Immobilisierung erfolgt auf einer Trägeroberfläche, wobei der Träger aus Glas, Polymer, Silica oder Nanopartikeln besteht.

### Ausführungsbeispiel 4: Darstellung eines modifizierten Polygycerols

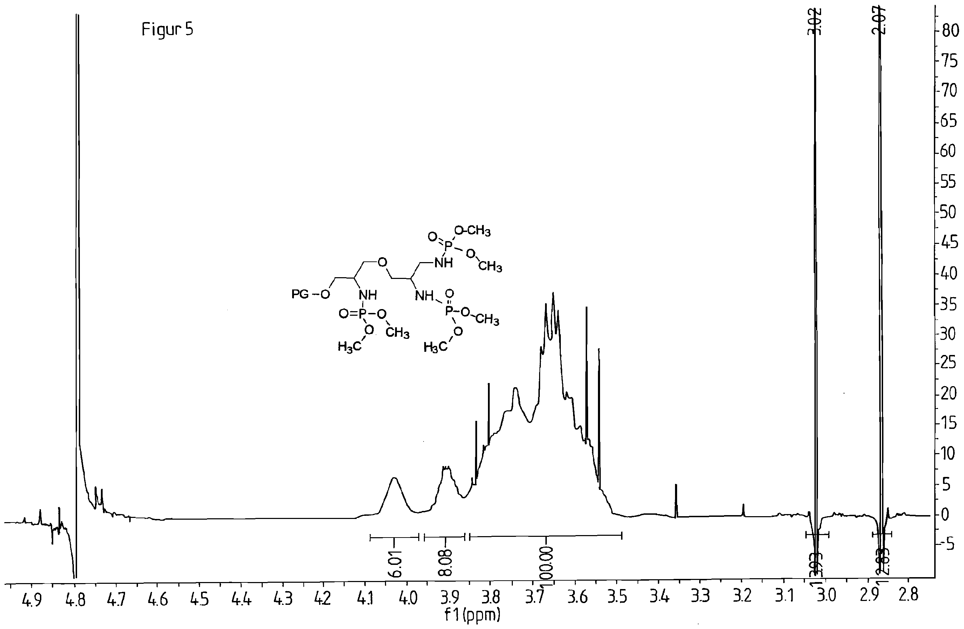

Ein azidhaltiges Polyglycerol mit einem durchschnittlichen Molekulargewicht von 10,000 g mol-1 (PG10) wird mit einem leichten Überschuss an Trimethylphosphit umgesetzt, wobei ein komplette Umsetzung zum Methylphosphoramidat-Derivat erfolgt. Das 1H-NMR Spektrum (Figur 5) zeigt durch Doublets mit einer Kopplungskonstante von jeweils 12 Hz eindeutig, dass Methylphosphoramidateinheiten vorliegen (Kupplung von Methylprotonen mit Phosphor).

### Ausführungsbeispiel 5: Darstellung von Phosphonamid-haltigen Peptiden durch eine Staudinger-Phosphonit Reaktion

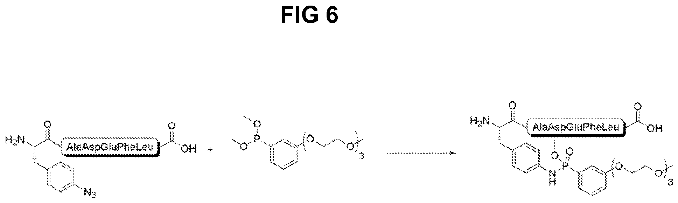

Die chemoselektive Funktionalisierung von Azido-Peptiden mit einer Phosphonamid-Einheit wurde durch folgende Experimente gezeigt. Ein mit einer Polyethylenglycolmonomethylether-Gruppe funktionalisiertes Arylphosphonit, das synthetisch über drei Stufen zugänglich ist, wurde einem Azido-Peptid, das ein Arylazid trägt, in Tris/HCl Puffer bei pH=8.2 für 30 Minuten umgesetzt (Figur 6).

**Tabelle 3: Verwendung von Phosponiten zur Modifizierung von Peptiden**

| **Phosphonit (Äq.)** | **Reaktionsbedingungen (25 °C, 30 min)** | **Azidopeptid (Startmaterial)^{a}** | **Phosphonamide (Produkt)^{a}** |
|---|---|---|---|
| R² = Me (12 eq.) | 1M Tris/HCl Puffer (pH 8.2) | 52% | 48% |
| R² = Me (100 eq.) | 1 M Tris/HCl Puffer (pH 8.2) | 43% | 57% |
| R² = Me (100 eq.) | 1 M Tris/HCl Puffer (pH 8.2) w/ 10 % DMSO | < 1% | > 99% |

| | | | |
|---|---|---|---|
| a Quantifizierung durch HPLC-MS | | | |

In der anschließenden Analyse des Reaktionsgemisches mittels HPLC-MS konnte neben dem gewünschten Phosphonamid-haltigem Peptid lediglich noch das nicht-umgesetzte Startmaterial des Azido-Peptides nachgewiesen werden. Eine Erhöhung der Phosphonitäquivalente sowie das Beimischen von 10% DMSO zur Verbesserung der Löslichkeit und der Stabilität des Phosphonits, führten zu einer nahezu vollständigen Umsetzung des Azido-Peptids zum Phosphonamid-haltigen Peptiden (siehe Figur 6). Zusammenfassend kann daher festgehalten werden, dass die Staudinger-Phosphonit-Reaktion zur Darstellung von funktionalisierten Phosphonamid-haltigen Proteinen verwendet werden kann (Vallee, Hackenberger, unveröffentlichte Ergebnisse)

### Ausführungsbeispiel 6: PEGylierung von Proteinen mit PEG-haltigen Phosphiten

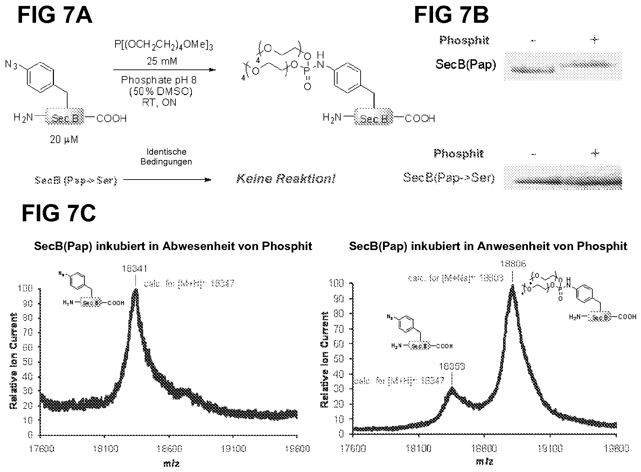

Die chemoselektive PEGylierung von Proteinen, wurde mit dem Protein SecB durchgeführt, das durch unnatürliche Protein Exprimierung gewonnen wurde und eine Arylazideinheit trägt (hier als SecB(Pap) bezeichnet, siehe auch Serwa, Hackenberger et al., Angew. Chem. 2009, 48:8234-8239). Daraufhin wurde das Protein mit einem symmetrischen Phosphit umgesetzt (Figur 7A), das jeweils vier PEG-Einheiten trägt (Serwa, Hackenberger, et. al. unveröffentlichte Ergebnisse). Der Reaktionsumsatz wurde daraufhin durch Gelelektrophorese untersucht (Figur 7B). Dabei stellte sich heraus, dass nach 14 Stunden Reaktionsführung ein quantitativer Umsatz von dem SecB(Pap) stattfand, da ein vollständiger Gelshift beobachtet wurde (Figur 7, oben). In einem Kontrollexperiment, bei dem die Azideinheit gegen ein Serin ausgetauscht wurde, konnte dagegen kein Gelshift beobachtet werden (Figur 7B unten), was die Chemoselektivität der PEGylierung demonstriert.

Ein weiterer Beweis für die chemoselektive Phosphorylierung wurde durch eine MALDI-MS Analyse vorgenommen (Figur 7C). Dabei konnte nur beim Azidopeptide SecB(Pap) ein kovalentes Massenadukt beobachtet werden (Figur 7C rechts). Das Vergleichsexperiment mit dem Azidfreien Protein lieferte kein Massenadukt (Figur 7C links).

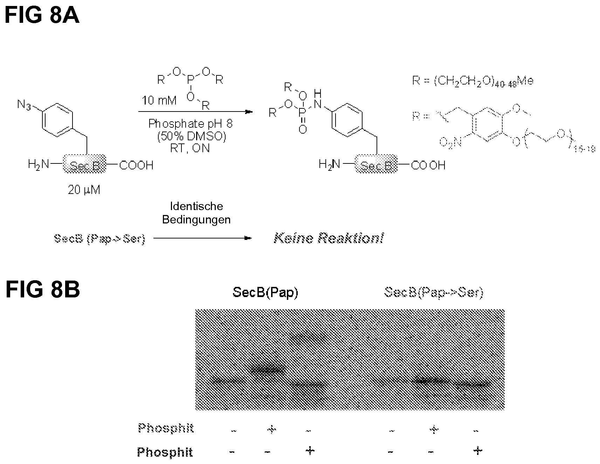

Anschließend wurde das SecB(Pap) auch mit zwei längeren PEG-Phosphiten mit 40-48 PEG Einheiten pro Phosphit-Substituent bzw. 15-19 Einheiten umgesetzt (Figur 8A). Auch hier zeigten sich entsprechende chemoselektive PEGylierungen wie durch Gel-Elektrophorese verifiziert (Figur 8B).

Zusammenfassend lässt sich feststellen, dass verschiedene PEG-haltige Phosphite für quantitative Umsetzungen von Proteinen verwendet werden können. Die Reaktionen laufen und milden Bedingungen (Raumtemperatur) in wässrigen Puffersystemen ab. Neben entsprechenden Phosphoramidat-PEG Bausteinen konnte auch ein lichtspaltbares PEG-Phosphit hergestellt werden (grünes Phosphit in Figur 8), bei dem die PEG Einheit durch Laserpuls wieder entfernt werden kann, was für schaltbare Proteinsysteme sehr vielversprechend sein kann.

### Ausführungsbeispiel 7: Biotinylierung von Proteinen mit einem unsymmetrischen Biotin-Phosphit

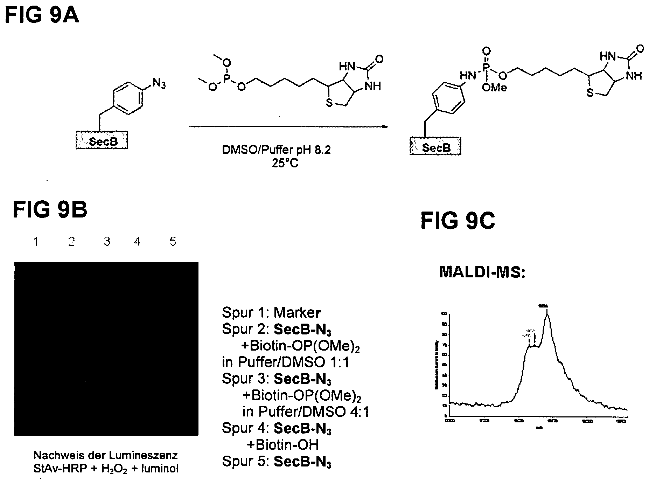

Die chemoselektive Konjugation von Biotin-haltigen Phosphiten wurde ebenfalls mit dem Azid-haltigen Modellprotein SecB demonstriert. Dazu wurde ein unsymmetrisches Biotinphosphit über 3 Stufen synthetisch hergestellt und mit dem Azidoprotein in wässrigem Puffer mit DMSO umgesetzt (Figur 9A). Eine Analyse über Gelelektrophorese und MALDI-MS (Figur 9B und 9C) zeigte verifizierte die Konjugation über die Staudinger-Phosphit Reaktion (Böhrsch, Hackenberger et al, Chem. Commun. 2010, zur Veröffentlichung angenommen).

## Patentansprüche

1. Modifiziertes, mindestens eine weitere funktionelle Gruppe aufweisendes Makromolekül, **gekennzeichnet durch** mindestens eine x-fach (x≥1) chemoselektiv eingebaute Phosphoramidat-Gruppe der allgemeinen Formel (I)
-NHPO(OR¹)(OR^{1'}) (I),
und/oder mindestens eine x-fach (x≥1) chemoselektiv eingebaute Phosphonamid-Gruppe der allgemeinen Formel (Ia)
-NHPO(R¹)(OR^{1'}) (Ia)
wobei R¹ und R^{1'} ausgewählt ist aus der Gruppe enthaltend Glycerol, Polyglycerol, PEG-Polymere der allgemeinen Summenformel C₂ₙH₄ₙ₊₂Oₙ₊₁ mit n ≥ 1, Cₙ-Alkylketten mit n ≥ 1; funktionalisierte Cₙ-Alkylketten mit n ≥ 1, Aryle, Heteroaryl, Silyl, Lipide, Fluorophore, Saccharide, Peptide, Kronenether, oder ein Linker, der die oben angegebenen Gruppen verknüpft, und wobei R¹ und R^{1'} gleich oder verschieden voneinander sein können, wobei die mindestens eine weitere funktionelle Gruppe ausgewählt ist aus der Gruppe enthaltend -OH, -NH₂, -SH, -CO₂H, -CONH₂, sowie Disulfide, Guanidine, Imidiazole, Indole, Alkene und/oder Alkine, und wobei die in dem Makromolekül vorliegende weitere funktionelle Gruppe ungeschützt vorliegt.

2. Makromolekül nach Anspruch 1, **dadurch gekennzeichnet, dass** die Anzahl x der eingebauten Phosphoramidat- und/oder Phosphonsäuregruppen zwischen 1 und 50, besonders bevorzugt zwischen 1 und 30 und ganz besonders bevorzugt zwischen 1 und 15, liegt.

3. Makromolekül nach Anspruch 1 oder 2, **dadurch gekennzeichnet, dass** die Phosphoramidatgruppe der allgemeinen Formel (I) in eine Phosphoramid-Gruppe der allgemeinen Formel (III)
-NHPO(OH)₂ (III)
und/oder dass die Phosphonamidgruppe der allgemeinen Formel (la) in eine freie Phosphonamid-Gruppe der allgemeinen Formel (IIIa)
-NHP(O)R¹(OH)
umwandelbar ist.

4. Makromolekül nach Anspruch 1 oder 2, **dadurch gekennzeichnet, dass** das über die Phosphoramidat- und/oder Phosphonamidgruppe auf der Oberfläche eines Trägers immobilisierbar ist.

5. Makromolekül nach einem der vorhergehenden Ansprüche, **dadurch gekennzeichnet, dass** als Makromolekül Polymere, Peptide mit einer Kettenlänge von größer 6 Aminosäuren, Proteine, Glykane, Lipide, Polynukleotide und/oder Polyketide verwendet werden.

6. Verwendung des Makromoleküls nach einem der vorhergehenden Ansprüche als Phosphatase- oder Kinasesubstrate, als Markermoleküle, als biochemische Sonden zur Aufklärung biologischer Funktion, als Stabilisierungsfaktoren gegenüber Proteolyse durch den Einbau von PEG oder als pharmazeutisch wirksame Substanzen, insbesondere Inhibitoren.

7. Verfahren zur chemoselektiven Phosphorylierung von Makromolekülen umfassend die folgenden Schritte:
a) Synthese und/oder Funktionalisierung eines mindestens eine funktionelle Gruppe aufweisendes Makromolekül mit mindestens einer Azid (N₃) - Gruppe, bevorzugt mit einer Alkyl- und/oder Arylazidgruppe, wobei das Makromolekül mindestens eine weitere ungeschützt funktionelle Gruppe ausgewählt aus der Gruppe enthaltend -OH, -NH₂, -SH, -CO₂H, -CONH₂ sowie Disulfide, Guanidine wie -NHCNHNH₂ in Arginin, Imidiazole wie in Histidin, Indole wie in Tryptophan, Alkene und/oder Alkine aufweist ,und
b) Umsetzung der mindestens einen Azid-Gruppe des Makromoleküls
- mit einem Phosphit der allgemeinen Formel (II) P(OR¹)(OR^{1'})(OR^{1"}) unter Ausbildung einer Phosphoramidat-Gruppe der allgemeinen Formel (I) -NHPO(OR¹)(OR^{1'}) und/oder
- mit einem Phosphonit der allgemeinen Formel (IIa) PR¹(OR^{1'})(OR^{1"}) unter Ausbildung einer Phosphonamid-Gruppe der allgemeinen Formel (la) -NHPO(R¹)(OR^{1'}), wobei den Resten R¹, R^{1'} und R^{1"} die obige Bedeutung von R¹ und R^{1'} zukommt.

8. Verfahren nach Anspruch 7, **dadurch gekennzeichnet, dass** in einem sich an den Schritt b) anschließendem Schritt c) die Phosporamidatgruppe der allgemeinen Formel (I) zu einer Phosphoramid-Gruppe der allgemeinen Formel (III) -NHPO(OH)₂ und/oder die Phosphonamidgruppe der allgemeinen Formel (la) zu einer Phosphonamid-Gruppe der allgemeinen Formel (IIIa) -NHPO(R¹)OH umgesetzt wird.

9. Verfahren nach Anspruch 8, **dadurch gekennzeichnet, dass** der Schritt der Umsetzung der Phosporamidat- und/oder Phosphonamidgruppe photolytisch und/oder hydrolytisch erfolgt.

10. Verfahren zur ortsspezifischen Immobilisierung von Makromolekülen umfassend die folgenden Schritte
- Synthese oder Funktionalisierung eines mindestens eine funktionelle Gruppe aufweisendes Makromoleküls umfassend mindestens eine Azid (N₃) - Gruppe, wobei das Makromolekül mindestens eine weitere ungeschützte funktionelle Gruppe ausgewählt aus der Gruppe enthaltend -OH, -NH₂, -SH, -CO₂H, -CONH₂ sowie Disulfide, Guanidine wie -NHCNHNH₂ in Arginin, Imidiazole wie in Histidin, Indole wie in Tryptophan, Alkene und/oder Alkine aufweist,
- Kopplung von mindestens einem Phosphit der allgemeinen Formel IV P(OR²)(OR^{2'})(OR^{2"}) und/oder mindestens einem Phosphonit der allgemeinen Formel (IVa) PR²(OR^{2'})(OR^{2"}) an die Oberfläche von mindestens einem Träger, wobei R², R^{2'} und R^{2"} ausgewählt sind aus der Gruppe enthaltend Cₙ-Alkylketten mit n ≥ 1, funktionalisierte Cₙ-Alkylketten mit n ≥ 1, Aryle, Heteroaryl, oder Silyl, wobei R², R^{2'} und R^{2"} gleich oder verschieden voneinander sein können, und
- Umsetzung der mindestens einen Azid-Gruppe des Makromoleküls mit dem mindestens einen mit einem Phosphit und/oder Phosphonit gekoppelten Träger, wobei eine Kopplung zwischen dem Träger und dem Makromolekül über eine Phosphoramidat- und/oder Phosphonamidgruppe erfolgt.

11. Verfahren nach Anspruch 10, **dadurch gekennzeichnet, dass** als Träger funktionalisierte Glasoberflächen, Mikroplatten, Nanopartikel, insbesondere Gold oder Silicananopartikel und Quantum Dots verwendet werden.

12. Träger herstellbar nach einem Verfahren gemäß Anspruch 10 oder 11.

## Claims

1. A modified macromolecule that has at least one further functional group, **characterized by** at least one x-fold (x ≥ 1) chemoselectively incorporated phosphoramidate group of general formula (I)
-NHPO(OR¹)(OR^{1'}) (I) ,
and/or at least one x-fold (x ≥ 1) chemoselectively incorporated phosphonamide group of general formula (Ia)
-NHPO(R¹)(OR^{1'}) (Ia)
wherein R¹ and R^{1'} is selected from the group containing glycerol, polyglycerol, PEG polymers of the general empirical formula C₂H₄ₙ₊₂Oₙ₊₁ with n ≥ 1, Cₙ-alkyl chains with n ≥ 1; functionalized Cₙ-alkyl chains with n ≥ 1, aryls, heteroaryl, silyl, lipids, fluorophores, saccharides, peptides, crown ethers, or a linker, which links the aforementioned groups, and wherein R¹ and R^{1'} can be identical to or different from one another, wherein the at least one is further functional group is selected from a group containing -OH, -NH₂, -SH, -CO₂H, -CONH₂, disulfides, guanidines, imidazoles, indoles, alkenes and/or alkynes, and wherein the further functional group of the modified macromolecule is in unprotected form.

2. The macromolecule as claimed in claim 1, **characterized in that** the number x of incorporated phosphoramidate and/or phosphonic acid groups is between 1 and 50, especially preferably between 1 and 30 and quite especially preferably between 1 and 15.

3. The macromolecule as claimed in claim 1 or 2, **characterized in that** the phosphoramidate group of general formula (I) can be converted to a phosphoramide group of general formula (III)
-NHPO(OH)₂ (III)
and/or **in that** the phosphonamide group of general formula (Ia) can be converted to a free phosphonamide group of general formula (IIIa)
-NHP(O)R¹(OH).

4. The macromolecule as claimed in claim 1 or 2, **characterized in that** the macromolecule can be immobilized via the phosphoramidate and/or phosphonamide group on the surface of a support.

5. The macromolecule as claimed in one of the preceding claims, **characterized in that** polymers, peptides with a chain length of greater than 6 amino acids, proteins, glycans, lipids, polynucleotides and/or polyketides are used as macromolecule.

6. A use of the macromolecule as claimed in one of the preceding claims as phosphatase or kinase substrates, as marker molecules, as biochemical probes for elucidating biological function, as stabilizing factors against proteolysis by incorporation of PEG or as pharmaceutically active substances, in particular inhibitors.

7. A method of chemoselective phosphorylation of macromolecules comprising the following steps:
a) synthesis and/or functionalization of a macromolecule having at least one functional group with at least one azide (N₃) group, preferably with an alkyl and/or arylazide group, wherein the azide has at least one further functional group which is in unprotected form selected from the group containing -OH, -NH₂, -SH, -CO₂H, -CONH₂ as well as disulfides, guanidines such as -NHCNHNH₂ in arginine, imidazoles as in histidine, indoles as in tryptophan, alkenes and/or alkynes, and
b) reaction of the at least one azide group of the macromolecule
- with a phosphite of general formula (II) P(OR¹)(OR^{1'})(OR^{1"}) with formation of a phosphoramidate group of general formula (I) -NHPO(OR¹)(OR^{1'}) and/or
- with a phosphonite of general formula (IIa) PR¹(OR^{1'})(OR^{1"}) with formation of a phosphonamide group of general formula (Ia) -NHPO(R¹)(OR^{1'}), wherein the moieties R¹, R^{1'} and R^{1"} are assigned the above meaning of R¹ and R^{1'}.

8. The method as claimed in claim 7, **characterized in that** in a step c) following step b), the phosphoramidate group of general formula (I) is reacted to a phosphoramide group of general formula (III)-NHPO(OH)₂ and/or the phosphonamide group of general formula (Ia) is reacted to a phosphonamide group of general formula (IIIa) -NHPO(R¹)OH.

9. The method as claimed in claim 8, **characterized in that** the step of reaction of the phosphoramidate and/or phosphonamide group takes place by photolysis and/or hydrolysis.

10. A method for site-specific immobilization of macromolecules comprising the following steps
- synthesis or functionalization of a macromolecule having at least one functional group comprising at least one azide (N₃) group, wherein the azide has at least one further functional group which is in unprotected form selected from the group containing -OH, -NH₂, -SH, -CO₂H, -CONH₂ as well as disulfides, guanidines such as -NHCNHNH₂ in arginine, imidazoles as in histidine, indoles as in tryptophan, alkenes and/or alkynes,
- coupling of at least one phosphite of general formula IV P(OR²)(OR^{2'})(OR^{2"}) and/or at least one phosphonite of general formula (IVa) PR²(OR^{2'})(OR^{2"}) on the surface of at least one support, wherein R², R^{2'} and R^{2"} are selected from the group containing Cₙ-alkyl chains with n ≥ 1, functionalized Cₙ-alkyl chains with n ≥ 1, aryls, heteroaryl, or silyl, wherein R², R^{2'} and R^{2"} can be identical to or different from one another, and
- reaction of the at least one azide group of the macromolecule with the at least one support coupled to a phosphite and/or phosphonite, wherein coupling between the support and the macromolecule takes place via a phosphoramidate and/or phosphonamide group.

11. The method as claimed in claim 10, **characterized in that** functionalized glass surfaces, microplates, nanoparticles, in particular gold or silica nanoparticles and quantum dots are used as support.

12. A support that can be made by a method as claimed in claim 10 or 11.

## Revendications

1. Macromolécule modifiée, comprenant au moins un groupe fonctionnel supplémentaire, **caractérisée par** au moins un groupe phosphoramidate de formule générale (I) incorporé chimiosélectivement x fois (x ≥ 1)
-NHPO(OR¹)(OR^{1'}) (I)
et/ou au moins un groupe phosphonamide de formule générale (Ia) incorporé chimiosélectivement x fois (x ≥ 1)
⁻NHPO(R¹)(OR^{1'}) (Ia)
dans laquelle R¹ et R^{1'} sont choisis dans le groupe contenant le glycérol, le polyglycérol, les polymères de PEG de formule globale générale C₂ₙH₄ₙ₊₂Oₙ₊₁ avec n ≥ 1, les chaînes alkyle en Cₙ avec n ≥ 1 ; les chaînes alkyle en Cₙ fonctionnalisées avec n ≥ 1, les aryles, les hétéroaryles, les silyles, les lipides, les fluorophores, les saccharides, les peptides, les éthers couronnes ou un agent de liaison, qui relie les groupes indiqués précédemment, R¹ et R^{1'} pouvant être identiques ou différents l'un de l'autre, ledit au moins un groupe fonctionnel supplémentaire étant choisi dans le groupe contenant -OH, -NH₂, -SH, -CO₂H, -CONH₂, ainsi que les disulfures, les guanidines, les imidazoles, les indoles, les alcènes et/ou les alcynes, et le groupe fonctionnel supplémentaire présent dans la macromolécule se présentant sous forme non protégée.

2. Macromolécule selon la revendication 1, **caractérisée en ce que** le nombre x de groupes phosphoramidate et/ou acide phosphonique incorporés est compris entre 1 et 50, de manière particulièrement préférée entre 1 et 30 et de manière tout particulièrement préférée entre 1 et 15.

3. Macromolécule selon la revendication 1 ou 2, **caractérisée en ce que** le groupe phosphoramidate de formule générale (I) peut être transformé en un groupe phosphoramide de formule générale (III)
-NHPO(OH)₂ (III)
et/ou **en ce que** le groupe phosphonamide de formule générale (Ia) peut être transformé en un groupe phosphonamide libre de formule générale (IIIa)
-NHP(O)R¹(OH).

4. Macromolécule selon la revendication 1 ou 2, **caractérisée en ce qu'**elle peut être immobilisée sur la surface d'un support par le biais du groupe phosphoramidate et/ou phosphonamide.

5. Macromolécule selon l'une quelconque des revendications précédentes, **caractérisée en ce que** des polymères, des peptides ayant une longueur de chaîne supérieure à 6 acides aminés, des protéines, des glycanes, des lipides, des polynucléotides et/ou des polycétides sont utilisés en tant que macromolécule.

6. Utilisation de la macromolécule selon l'une quelconque des revendications précédentes en tant que substrats de phosphatase ou de kinase, en tant que molécules de marquage, en tant que sondes biochimiques pour la clarification d'une fonction biologique, en tant que facteurs de stabilisation contre la protéolyse par l'incorporation de PEG ou en tant que substances pharmaceutiquement actives, notamment inhibiteurs.

7. Procédé de phosphorylation chimiosélective de macromolécules comprenant les étapes suivantes :
a) la synthèse et/ou la fonctionnalisation d'une macromolécule comprenant au moins un groupe fonctionnel avec au moins un groupe azide (N₃), de préférence avec un groupe alkyle et/ou arylazide, la macromolécule comprenant au moins un groupe fonctionnel non protégé supplémentaire choisi dans le groupe contenant -OH, -NH₂, -SH, -CO₂H, -CONH₂, ainsi que les disulfures, les guanidines, telles que -NHCNHNH₂ dans l'arginine, les imidazoles tels que dans l'histidine, les indoles tels que dans le tryptophane, les alcènes et/ou les alcynes, et
b) la mise en réaction dudit au moins un groupe azide de la macromolécule
- avec un phosphite de formule générale (II) P(OR¹)(OR^{1'})(OR^{1"}) avec formation d'un groupe phosphoramidate de formule générale (I)
-NHPO(OR¹)(OR^{1'})
et/ou
- avec un phosphonite de formule générale (IIa) PR¹(OR^{1'})(OR^{1"}) avec formation d'un groupe phosphonamide de formule générale (Ia)
-NHPO(R¹)(OR^{1'}),
les radicaux R¹, R^{1'} et R^{1"} ayant la signification précédente de R¹ et R^{1'}.

8. Procédé selon la revendication 7, **caractérisé en ce que,** lors d'une étape c) suivant l'étape b), le groupe phosphoramidate de formule générale (I) est transformé en un groupe phosphoramide de formule générale (III) - NHPO(OH₂) et/ou le groupe phosphonamide de formule générale (Ia) est transformé en un groupe phosphonamide de formule générale (IIIa) -NHPO(R¹)OH.

9. Procédé selon la revendication 8, **caractérisé en ce que** l'étape de transformation du groupe phosphoramidate et/ou phosphonamide a lieu photolytiquement et/ou hydrolytiquement.

10. Procédé d'immobilisation spécifique au lieu de macromolécules comprenant les étapes suivantes :
- la synthèse ou la fonctionnalisation d'une macromolécule comprenant au moins un groupe fonctionnel comprenant au moins un groupe azide (N₃), la macromolécule comprenant au moins un groupe fonctionnel non protégé supplémentaire choisi dans le groupe contenant -OH, -NH₂, -SH, -CO₂H, -CONH₂, ainsi que les disulfures, les guanidines, telles que -NHCNHNH₂ dans l'arginine, les imidazoles tels que dans l'histidine, les indoles tels que dans le tryptophane, les alcènes et/ou les alcynes,
- le couplage d'au moins un phosphite de formule générale IV P(OR²)(OR^{2'})(OR^{2"}) et/ou d'au moins un phosphonite de formule générale (IVa) PR²(OR^{2'})(OR^{2"}) sur la surface d'au moins un support, R², R^{2'} et R^{2"} étant choisis dans le groupe contenant les chaînes alkyle en Cₙ avec n ≥ 1, les chaînes alkyle en Cₙ fonctionnalisées avec n ≥ 1, les aryles, les hétéroaryles ou les silyles, R², R^{2'} et R^{2"} pouvant être identiques ou différents les uns des autres, et
- la mise en réaction dudit au moins un groupe azide de la macromolécule avec ledit au moins un support couplé avec un phosphite et/ou un phosphonite, un couplage ayant lieu entre le support et la macromolécule par le biais d'un groupe phosphoramidate et/ou phosphonamide.

11. Procédé selon la revendication 10, **caractérisé en ce que** des surfaces de verre fonctionnalisées, des microplaques, des nanoparticules, notamment des nanoparticules d'or ou de silice, et des points quantiques sont utilisés en tant que support.

12. Support pouvant être fabriqué par un procédé selon la revendication 10 ou 11.
